# EUROPÄISCHE PATENTSCHRIFT

(11) **EP 0 367 836 B1**
(45) Veröffentlichungstag und Bekanntmachung des Hinweises auf die Patenterteilung: **20.01.1993**
(21) Anmeldenummer: 88117043.5
(22) Anmeldetag: 13.10.1988
(51) Int. Cl.: A61B 6/00

(54) **Fahrbares Röntgendiagnostikgerät mit einer höhenverstellbaren Säule**
Mobile X-ray diagnostic apparatus with an adjustable elevation for the column
Appareil diagnostic à rayons X mobile avec une colonne ajustable à élévation

(43) Veröffentlichungstag der Anmeldung: 16.05.1990
(73) Patentinhaber: SIEMENS AKTIENGESELLSCHAFT, 80333 München (DE)
(72) Erfinder: Kaul, Karlheinz, Dipl.-Ing. (FH), D-8525 Weiher (DE); Bock, Hans-Christian, Dipl.-Ing. (FH), D-8525 Uttenreuth (DE); Saffer, Edmund, D-8551 Eggolsheim (DE)

(56) Entgegenhaltungen:
- DE-B- 2 640 644
- US-A- 2 168 209
- US-A- 4 387 468

## Beschreibung

Die Erfindung betrifft ein fahrbares Röntgendiagnostikgerät mit einer an einem Fahrgestell einseitig gelagerten, höhenverstellbaren Säule, die über eine Halterung einen Röntgenstrahler und einen Strahlenempfänger trägt.

Ein solches Röntgendiagnostikgerät ist beispielsweise aus der DE-A-1 958 905 bekannt. Bei diesem Röntgendiagnostikgerät ist die Säule über einen elektromotorischen Antrieb in der Höhe verstellbar.

Zur Höhenverstellung der Säule ist dem elektromotorischen Antrieb die elektrische Energie aus dem Netz oder eines Akkus zuzuführen. Zur Spannungsversorgung aus dem Netz ist eine Leitung und ein Energieversorgungsanschluß, zu dem die Leitung führt, vorzusehen. Bei der Energieversorgung aus einem Akku ist das Fahrgestell des Röntgendiagnostikgerätes entsprechend dem höheren Gewicht und den Abmessungen des Akkus auszulegen.

Aus der US-A-2 168 209 ist eine Konstruktion bekannt, die eine vertikale, starre Säule aufweist, an der ein Wagen höhenverstellbar ist, der einen Röntgenstrahler trägt. Am Wagen greift eine vertikal in der Säule angeordnete Feder über ein Seil, daß um verschiedene Umlenkungen geführt ist, an, so daß dieser mit dem Röntgenstrahler gewichtsausgeglichen verstellbar ist.

Aufgabe der Erfindung ist es daher, ein Röntgendiagnostikgerät der eingangs genannten Art so auszuführen, daß eine Höhenverstellung der Säule bei einfachem, platzsparendem Aufbau leicht ohne einen elektromotorischen Antrieb möglich ist.

Die Aufgabe ist erfindungsgemäß dadurch gelöst, daß ein Ende eines Seiles mit der Säule im Bereich ihres unteren Endes verbunden ist und daß das Seil derart über eine Umlenkung geführt ist, so daß es auf die Säule eine vertikale Gewichtsausgleichskraft ausübt, welche von einer Feder erzeugt wird, die horizontal in einem Ausleger des Fahrgestelles des Röntgendiagnostikgerätes gespannt ist.

Vorteil der Erfindung ist, daß die Säule gewichtsausgeglichen höhenverstellbar ist und daß hierzu keine elektrische Energie notwendig ist. Dies ist insbesondere beim Verfahren des Röntgendiagnostikgerätes auf einer Station vorteilhaft, wenn die Säule mit dem Röntgenstrahler und dem Strahlenempfänger nach dem Anfertigen einer Röntgenaufnahme nicht sofort in die niedrigste Stellung gefahren wurde. Beim Stand der Technik muß dann das Kabel zur Energieversorgung mit einem Energieversorgungsanschluß verbunden werden, um die Säule in ihre unterste Stellung zu fahren. Ein solcher Anschluß ist aber unter Umständen nicht in erreichbarer Nähe, so daß das Röntgendiagnostikgerät zu einem solchen Anschluß gefahren werden muß, bevor die eigentliche Fahrt fortgesetzt werden kann. Dahingegen kann die Säule bei einem Röntgendiagnostikgerät nach der Erfindung ohne das Zuführen von elektrischer Energie verstellt werden.

Gegenüber der US-A-2 168 209 ergibt sich durch das erfindungsgemäße fahrbare Röntgendiagnostikgerät und insbesondere durch die horizontal in dem Ausleger des Fahrgestelles angeordnete Feder sowie die Führung des Seiles ein erheblich kompakterer Aufbau.

Vorteilhaft ist, wenn eine Bremse zum Arretieren der Säule vorgesehen ist. Somit kann die Säule an jeder beliebigen Stelle des Verstellweges arretiert werden, insbesondere dann, wenn das Röntgendiagnostikgerät kippt.

Weitere Vorteile und Einzelheiten der Erfindung ergeben sich aus der nachfolgenden Beschreibung eines Ausführungsbeispieles anhand der Zeichnung in Verbindung mit den Unteransprüchen. Dabei zeigt die
- Figur 1: ein fahrbares Röntgendiagnostikgerät mit einer höhenverstellbaren Säule nach der Erfindung und
- Figur 2: eine Umlenkung mit einer spiralförmigen Trommel und einer Bremse zum Arretieren der Säule des Röntgendiagnostikgerätes gemäß Figur 1.

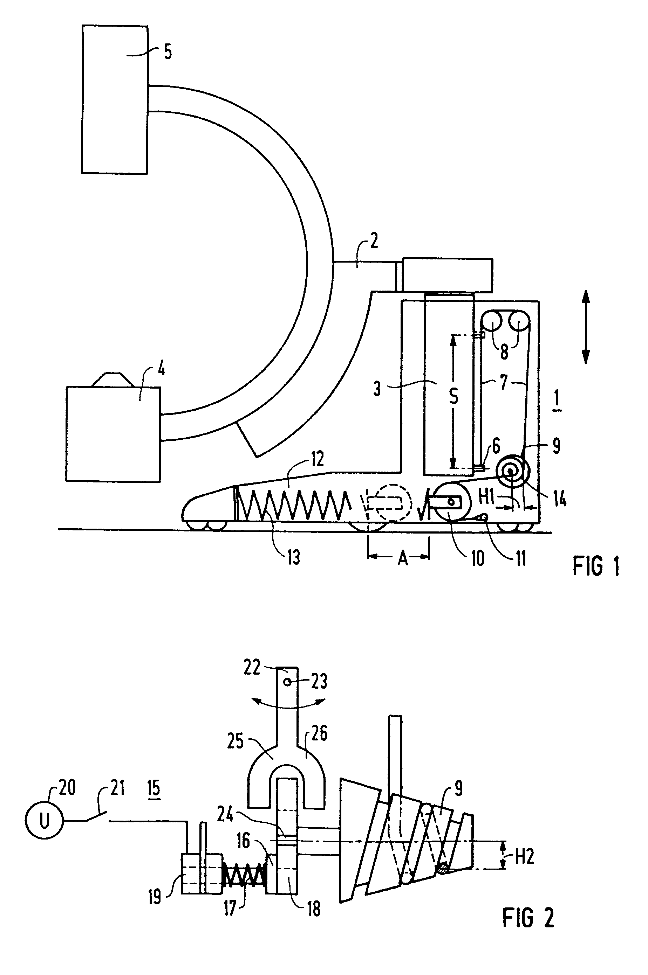

Die Figur 1 zeigt ein fahrbares Röntgendiagnostikgerät 1 mit einer Halterung 2, die über eine Säule 3 an einem Fahrgestell höhenverstellbar ist und die einen Röntgenstrahler 4 und einen Strahlenempfänger 5 trägt. Im unteren Bereich 6 der Säule 3 ist ein Seil 7 befestigt. Es ist über eine erste Umlenkung 8 mit zwei Rollen zu einer zweiten Umlenkung 9 geführt. Das Seil 7 ist um die Umlenkung 9, die als spiralförmige Trommel ausgebildet ist, gelegt und führt von dort über eine dritte Umlenkung 10 zu einer Befestigung 11 im unteren Bereich des Fahrgestelles. Die dritte Umlenkung 10 ist als Rolle ausgebildet und steht mit einer im Ausleger 12 des Fahrgestelles horizontal ausgerichteten vorgespannten Spiralfeder 13 in Verbindung. Durch die gespannte Spiralfeder 13 wirkt eine Zugkraft auf das Seil 7, die die Gewichtskraft der Säule 3 und der Halterung 2 mit dem Röntgenstrahler 4 und dem Strahlenempfänger 5 ausgleicht. Die Spirale 14 der zweiten Umlenkung 9 ist in Verbindung mit der Zugkraft der Spiralfeder 13 so ausgebildet, daß die Kraft zum Verstellen der Säule 3 über den gesamten Verstellweg S der Säule 3 gleichmäßig ist. Da sich die Zugkraft der Spiralfeder 13 in Abhängigkeit von deren Auszug A ändert, ist es notwendig, auch die Hebel H1, H2 (siehe auch Figur 2) der Umlenkung 9 bei einer Verstellung der Säule 3 so zu verändern, daß das Drehmoment der Umlenkung 9 und damit die Gewichtsausgleichskraft auf das Seil 7 konstant ist. Dies wird durch die Ausbildung der Spirale 14 erreicht. Beim Verstellen der Säule 3 längs des Verstellweges S rotiert die Spirale 14 um die Achse der Umlenkung 9. Dabei wird das Seil 7 entlang der konisch ausgebildeten Trommel geführt, wobei sich die wirksamen Hebel H1, H2 ändern.

Die Arretierung der Säule 3 erfolgt durch eine Bremse 15, die in der FIG 2 beispielhaft dargestellt ist. Sie besitzt ein Bremselement 16, das über die Kraft einer Spiralfeder 17 auf eine Bremsscheibe 18 gepreßt wird, die mit der Umlenkung 9 in Verbindung steht. Anstelle einer Spiralfeder 17 kann beispielsweise aber auch ein Permanentmagnet auf das Bremselement 16 wirken. Die Säule ist arretiert. Die Bremse wird gelöst, wenn einem elektromechanischen Antrieb 19 die Spannung einer Spannungsquelle 20 über einen Schalter 21 zugeführt wird. Dabei wird die Anpreßkraft des Bremselementes 16 aufgehoben, so daß die Bremsscheibe 18 und damit die Umlenkung 9 frei verstellbar ist. Die Anpreßkraft des Bremselementes 16 kann auch über nicht gezeigte Betätigungsmittel am Röntgendiagnostikgerät 1 aufgehoben werden. So kann die Bremse auch ohne das Zuführen von elektrischer Energie gelöst werden.

Ein gabelförmiges Element 22 ist über eine Lagerung 23 quer zur Bremsscheibe 18 verstellbar gelagert. Am Umfang der Bremsscheibe 18 sind Aussparungen 24 vorgesehen, in die das gabelförmige Element 22 mit einem der Schenkel 25 oder 26 beim seitlichen Verkippen des Röntgendiagnostikgerätes 1 eingreift, so daß die Verstellbarkeit der Säule 3 blockiert ist. Die Blockierung wird aufgehoben, wenn das Fahrgestell des Röntgendiagnostikgerätes 1 wieder annähernd horizontal ausgerichtet ist.

## Patentansprüche

1. Fahrbares Röntgendiagnostikgerät (1) mit einer an einem Fahrgestell einseitig gelagerten, höhenverstellbaren Säule (3), die über eine Halterung (2) einen Röntgenstrahler (4) und einen Strahlenempfänger (5) trägt, **dadurch gekennzeichnet,** daß ein Ende eines Seiles (7) mit der Säule (3) im Bereich (6) ihres unteren Endes verbunden ist und daß das Seil (7) derart über eine Umlenkung (8, 9, 10) geführt ist, so daß es auf die Säule (7) eine vertikale Gewichtsausgleichskraft ausübt, welche von einer Feder (13) erzeugt wird, die horizontal in einem Ausleger (12) des Fahrgestelles des Röntgendiagnostikgerätes (1) gespannt ist.

2. Röntgendiagnostikgerät nach Anspruch 1, **dadurch gekennzeichnet,** daß das Seil (7) vom unteren Bereich (6) der Säule (3) über mindestens eine obere Umlenkung (8), über eine zweite untere Umlenkung (9) und über eine dritte Umlenkung (10), die mit der Feder (13) in Verbindung steht, zu einer Befestigung des Seiles (7) am Fahrgestell geführt ist.

3. Röntgendiagnostikgerät nach Anspruch 1 oder 2, **dadurch gekennzeichnet**, daß das Seil (7) um eine als spiralförmige Trommel ausgebildete Umlenkung (9) geführt ist.

4. Röntgendiagnostikgerät nach einem der Ansprüche 1 bis 3, **dadurch gekennzeichnet,** daß im Bereich der Umlenkung (9) eine Bremse (15) vorgesehen ist zum Arretieren der Säule (3).

5. Röntgendiagnostikgerät nach Anspruch 4, **dadurch gekennzeichnet,** daß die Arretierung erfolgt, wenn das Röntgendiagnostikgerät (1) seitlich kippt.

## Claims

1. Mobile X-ray diagnostics apparatus (1) having a height-adjustable column (3) mounted on an undercarriage on one side, the column carrying an X-ray tube (4) and a radiation receiver (5) by way of a holder (2), characterized in that an end of a cable (7) is connected to the column (3) in a region (6) of its lower end and in that the cable (7) is guided by way of a deflection element (8, 9, 10) in such a way that it exerts on the column (7) a vertical weight-compensating force, which is generated by a spring (13) which is loaded horizontally in a cross-arm (12) of the undercarriage of the X-ray diagnostics apparatus (1).

2. X-ray diagnostics apparatus according to claim 1, characterized in that the cable (7) is guided from the lower region (6) of the column (3) to a fastening of the cable (7) on the undercarriage by way of at least one upper deflection element (8), by way of a second lower deflection element (9) and by way of a third deflection element (10) which is connected to the spring (13).

3. X-ray diagnostics apparatus according to claim 1 or 2, characterized in that the cable (7) is guided about a deflection element (9) constructed as a spiral drum.

4. X-ray diagnostics apparatus according to one of claims 1 to 3, characterized in that in the region of the deflection element (9) a brake (15) is provided for stopping the column (3).

5. X-ray diagnostics apparatus according to claim 4, characterized in that the stopping takes place when the X-ray diagnostics apparatus (1) tilts to the side.

## Revendications

1. Appareil de radiodiagnostic mobile (1) comportant une colonne (3) réglable en hauteur, qui est montée unilatéralement sur un châssis mobile et qui porte, par l'intermédiaire d'un support (2), un émetteur radiologique (4) et un récepteur de rayonnement (5), caractérisé par le fait qu'une extrémité d'un câble (7) est raccordé à la colonne (3) dans la zone (6) de son extrémité inférieure et que le câble (7) est guidé sur un système de renvoi (8,9,10) de sorte qu'il exerce sur la colonne (7) une force d'équilibrage verticale, qui est produite par un ressort (13), qui est tendu horizontalement dans un bras en console (12) du châssis mobile de l'appareil de radiodiagnostic (1).

2. Appareil de radiodiagnostic suivant la revendication 1, caractérisé par le fait que le câble (7) s'étend depuis la partie inférieure (6) de la colonne (3) en passant sur un élément de renvoi supérieur (8) et un élément de renvoi inférieur (9) et sur un troisième élément de renvoi (10), qui est raccordé au ressort (13), jusqu'à une fixation du câble (7) sur le châssis mobile.

3. Appareil de radiodiagnostic suivant la revendication 1 ou 2, caractérisé par le fait que le câble (7) circule autour d'un élément de renvoi (9) réalisé sous la forme d'un tambour spiral.

4. Appareil de radiodiagnostic suivant l'une des revendications 1 à 3, caractérisé par le fait qu'un frein (15) est prévu au niveau de l'élément de renvoi (9) pour bloquer la colonne (3).

5. Appareil de radiodiagnostic suivant la revendication 4, caractérisé par le fait que le blocage s'effectue lorsque l'appareil de radiodiagnostic (1) bascule latéralement.
